# FASCICULE DE BREVET EUROPEEN

(11) **EP 2 429 618 B1**
(45) Date de publication et mention de la délivrance du brevet: **08.02.2017**
(21) Numéro de dépôt: 09807449.5
(22) Date de dépôt: 31.12.2009
(51) Int. Cl.: A61B 90/11

(54) **DISPOSITIF PERMETTANT DE GUIDER LA SERINGUE DE L'UTILISATEUR POUR QU'IL PUISSE INJECTER SA SOLUTION ANALGESIQUE AU PLUS PRES DU FORAMEN MANDIBULAIRE**
VORRICHTUNG ZUR FÜHRUNG DER SPRITZE EINES ANWENDERS ZUR INJEKTION EINER SCHMERZSTILLENDEN LÖSUNG MÖGLICHST NAHE AM FORAMEN MANDIBULAE
DEVICE FOR GUIDING THE SYRINGE OF A USER SO AS TO ENABLE THE INJECTION OF THE ANALGESIC SOLUTION OF THE FORMER AS CLOSE TO THE MANDIBULAR FORAMEN AS POSSIBLE

(30) Priorité: 26.02.2009 FR 0900872
(43) Date de publication de la demande: 21.03.2012
(73) Titulaire: Caillieux, Nicolas, 10000 Troyes (FR)
(72) Inventeur: Caillieux, Nicolas, 10000 Troyes (FR)
(86) Numéro de dépôt international: PCT/FR2009/001493
(87) Numéro de publication internationale: WO 2010/097520

(56) Documents cités:
- WO-A-2005/065337
- DE-U1- 20 101 400
- GB-A- 2 356 143
- US-A1- 2009 048 610

## Description

La présente invention concerne un dispositif selon la revendication 1 permettant de guider la seringue de l'utilisateur pour qu'il puisse injecter sa solution analgésique au plus près du foramen mandibulaire lors de l'analgésie loco-régionale mandibulaire.

Le foramen mandibulaire est une structure anatomique dans laquelle s'insinue le nerf alvéolaire inférieur que l'on doit atteindre lorsque l'on effectue ce type d'analgésie. Ce foramen mandibulaire est situé sur la face interne de la branche mandibulaire mais il est masqué par deux obstacles anatomiques qui rendent difficile l'analgésie :
- un obstacle musculaire qui recouvre la face interne de la branche mandibulaire,
- un obstacle osseux appelé crête temporale sur lequel l'aiguille vient souvent buter avant d'atteindre le foramen.

L'analgésie régionale mandibulaire est habituellement réalisée en utilisant des repères osseux, dentaires (labiles) et musculaires qui tiennent compte d'indices anthropomorphiques moyens. Mais ils ne prennent pas en compte les variations interindividuelles de l'orientation sagittale de la branche mandibulaire, de sa largeur ni de l'angle qu'elle forme avec le corps de la mandibule ce qui accroît la difficulté rencontrée par les praticiens pour atteindre le foramen mandibulaire lors de la réalisation de ce type d'analgésie.

Le document WO-A-2005/065337 décrit un dispositif pour guider une seringue avec un gouttière de guidage.

Le dispositif selon l'invention permet de remédier à ces inconvénients
- en indiquant au praticien tous les axes qu'il peut donner à l'aiguille pour atteindre le foramen mandibulaire,
- lui permettant de choisir l'axe le plus haut et le plus latéral qu'il peut avoir dans la cavité orale pour éviter la crête temporale.

Les dessins annexés illustrent l'invention et la façon de l'utiliser :
La figure 1 représente le dispositif de l'invention.
La figure 2 représente une vue supérieure de la mandibule dont la branche mandibulaire droite a été sectionnée transversalement à hauteur du point (0) correspondant au foramen mandibulaire. Elle illustre la façon d'utiliser le dispositif.

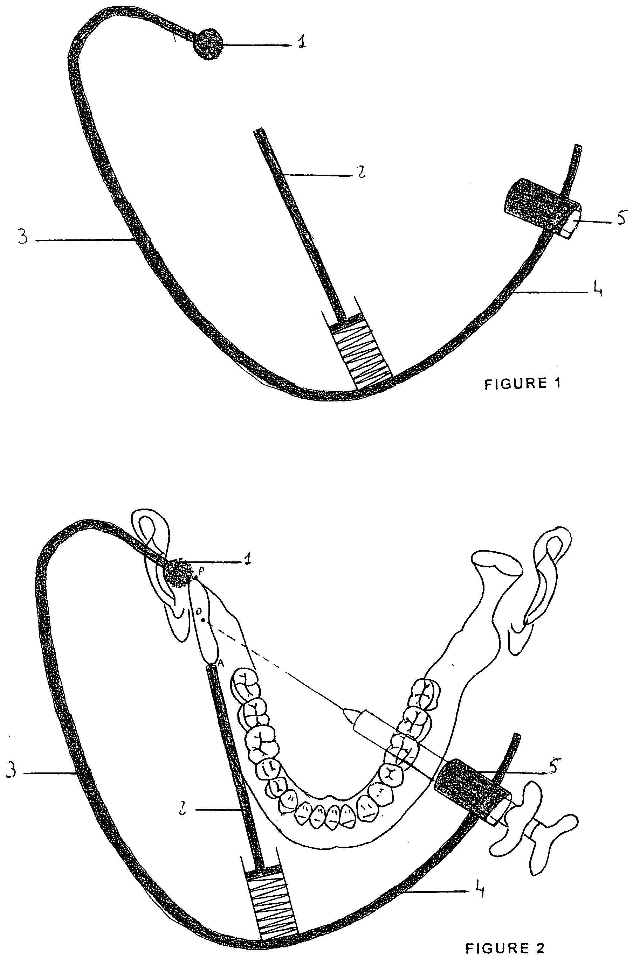

En référence à ces dessins, le dispositif de la figure 1 comporte un élément intermédiaire (3) permettant de relier à une de ses extrémités un embout auriculaire (1) et à son autre extrémité un élément rectiligne (2) ainsi qu'un élément curviligne (4). L'élément rectiligne (2) est positionné de telle sorte que son grand axe se projette sur l'élément (1). L'élément curviligne (4) est courbé de telle sorte qu'il présente un rayon égal à la distance entre le point de jonction des éléments (2) et (3) et l'élément (1) déduite de 1,5cm.
Un ressort fixé au point de jonction des éléments (2) et (3)
est destiné à pousser et à maintenir l'extrémité de l'élément (2).
L'élément (5) a une forme de gouttière qui coulisse le long de l'élément (4). Il est positionné de telle sorte que son grand axe coulisse perpendiculairement aux tangentes de l'élément (4). Il sert d'appui et de guide d'orientation pour la seringue.
Notons que la partie de l'élément (3) la plus proche de l'élément (1) est orientée en arrière et latéralement selon un axe correspondant à celui du conduit auditif externe.
La figure 2 illustre la façon d'utiliser le dispositif. Les éléments (1) (2) et (3) vont permettre d'enserrer la branche mandibulaire entre son bord antérieur (A) qui se confond avec l'extrémité de l'élément (2) et son bord postérieur (P) qui se confond avec l'élément (1) déterminant ainsi son orientation sagittale.
D'après le rayon de courbure de l'élément (4) centré sur le point (0) situé à environ 1,5cm du point P et l'orientation de l'élément (5) coulissant perpendiculairement aux tangentes de l'élément (4), tous les axes indiqués par l'élément (5) se projettent sur le point (O).

Le dispositif selon l'invention pourra être utilisé de la façon suivante. L'embout auriculaire (1) est introduit dans l'oreille du patient. Puis l'extrémité de l'élément (2) est appuyée en bouche contre le bord antérieur de la branche mandibulaire (A). L'utilisateur placera sa seringue dans la gouttière de l'élément (5) qu'il fera coulisser pour choisir l'axe permettant d'atteindre le foramen mandibulaire situé le plus haut et le plus latéral possible dans la cavité orale, ceci afin d'éviter de buter contre la crête temporale.

A titre d'exemple non limitatif, l'élément (2) aura une dimension de 15cm et le rayon de courbure de l'élément (4) sera de 20cm.

Le dispositif selon l'invention est particulièrement destiné aux Chirurgiens-Dentistes et aux Médecins Stomatologues réalisant des analgésies loco-régionales mandibulaires.

## Revendications

1. Dispositif permettant de guider la seringue de l'utilisateur pour qu'il puisse injecter sa solution analgésique au plus près du foramen mandibulaire lors de l'analgésie locorégionale mandibulaire qui comporte un élément intermédiaire (3) permettant de relier à une de ses extrémités un embout auriculaire (1) et à son autre extrémité un élément curviligne (4) prolongé par une gouttière de guidage (5), ainsi qu'un élément rectiligne (2).

2. Dispositif selon la revendication 1 **caractérisé en ce que** l'élément rectiligne (2) est positionné de telle sorte qu'il s'insère sur l'élément curviligne (4) et que son grand axe se projette sur l'embout auriculaire (1).

3. Dispositif selon la revendication 1 ou la revendication 2 **caractérisé en ce que** l'élément curviligne (4) est courbé de telle sorte qu'il présente un rayon égal à la distance entre le point de jonction de l'élément rectiligne (2) sur l'élément curviligne (4) et l'embout auriculaire (1) déduite de 1,5cm.

4. Dispositif selon l'une quelconque des revendications précédentes **caractérisé en ce qu'**un ressort fixé au point de jonction des éléments curviligne (4) et rectiligne (2) est destiné à pousser et à maintenir l'extrémité de l'élément rectiligne (2).

5. Dispositif selon l'une quelconque des revendications précédentes **caractérisé en ce que** la gouttière de guidage (5) a une forme de gouttière qui coulisse le long de l'élément curviligne (4).

6. Dispositif selon l'une quelconque des revendications précédentes **caractérisé en ce que** la gouttière de guidage (5) est positionné de telle sorte que son grand axe coulisse perpendiculairement aux tangentes de l'élément curviligne (4).

7. Dispositif selon l'une quelconque des revendications précédentes **caractérisé en ce que** la gouttière de guidage (5) sert d'appui et de guide d'orientation pour la seringue.

8. Dispositif selon l'une quelconque des revendications précédentes **caractérisé en ce que** tous les axes indiqués par la gouttière de guidage (5) se projettent sur un point (O) situé à environ 1,5cm en avant de l'élément embout auriculaire (1)

## Patentansprüche

1. Vorrichtung zum Führen der Spritze des Anwenders zur Injektion seiner analgetischen Lösung möglichst nahe am Foramen mandibulae bei der lokoregionalen mandibulären Analgesie, umfassend ein Zwischenelement (3), das das Befestigen eines aurikulären Ansatzstücks (1) an einem seiner Enden und eines gekrümmten Elements (4) an seinem anderen Ende erlaubt, verlängert durch eine Führungsrinne (5), sowie ein gerades Element (2).

2. Vorrichtung nach Anspruch 1, **dadurch gekennzeichnet, dass** das gerade Element (2) so angeordnet ist, dass es sich auf dem gekrümmten Element (4) einfügt und dass sich seine Hauptachse zu dem aurikulären Ansatzstück ersteckt (1).

3. Vorrichtung nach Anspruch 1 oder Anspruch 2, **dadurch gekennzeichnet, dass** das gekrümmte Element (4) so gebogen ist, dass es einen Radius gleich dem Abstand zwischen dem Verbindungspunkt des geraden Elements (2) auf dem gekrümmten Element (4) und dem aurikulären Ansatzstück (1) abzüglich 1,5 cm aufweist.

4. Vorrichtung nach einem der vorhergehenden Ansprüche, **dadurch gekennzeichnet, dass** eine am Verbindungspunkt des gekrümmten (4) und geraden (2) Elements befestigte Feder dazu bestimmt ist, das Ende des geraden Elements (2) zu drücken und zu halten.

5. Vorrichtung nach einem der vorhergehenden Ansprüche, **dadurch gekennzeichnet, dass** die Führungsrinne (5) eine Rinnenform aufweist, die entlang des gekrümmten Elements (4) verläuft.

6. Vorrichtung nach einem der vorhergehenden Ansprüche, **dadurch gekennzeichnet, dass** die Führungsrinne (5) so angeordnet ist, dass ihre Hauptachse senkrecht zu den Tangenten des gekrümmten Elements (4) verläuft.

7. Vorrichtung nach einem der vorhergehenden Ansprüche, **dadurch gekennzeichnet, dass** die Führungsrinne (5) als Stütze und Orientierungshilfe für die Spritze dient.

8. Vorrichtung nach einem der vorhergehenden Ansprüche, **dadurch gekennzeichnet, dass** sich alle durch die Führungsrinne (5) angegebenen Achsen zu einem Punkt (O) erstrecken, der sich etwa 1,5 cm vor dem aurikulären Ansatzstückelement (1) befindet.

## Claims

1. Device for guiding the syringe of a user so as to enable the injection of an analgesic solution as close to the mandibular foramen as possible during local-regional mandibular analgesia which comprises an intermediate element (3) for connecting to one of its ends an ear mould (1) and at its other end a curvilinear element (4) extended by a guide groove (5) and a rectilinear element (2).

2. Device according to claim 1, **characterised in that** the rectilinear element (2) is positioned such that it is inserted on the curvilinear element (4) and that its major axis projects onto the ear mould (1).

3. Device according to claim 1 or claim 2, **characterised in that** the curvilinear element (4) is curved such that it has a radius equal to the inferred distance between the junction point of the rectilinear element (2) on the curvilinear element (4) and the ear mould (1) of 1.5 cm.

4. Device according to any one of the preceding claims, **characterised in that** a spring fixed to the junction point of the curvilinear (4) and rectilinear (2) points is designed to push and hold the end of the rectilinear element (2).

5. Device according to any one of the preceding claims, **characterised in that** the guide groove (5) has a groove shape which slides along the curvilinear element (4).

6. Device according to any one of the preceding claims, **characterised in that** the guide groove (5) is positioned such that its major axis slides perpendicularly to the tangents of the curvilinear element (4).

7. Device according to any one of the preceding claims, **characterised in that** the guide groove (5) serves as a support and orientation guide for the syringe.

8. Device according to any one of the preceding claims, **characterised in that** all the axes indicated by the groove guide (5) project themselves on a point (O) located approximately 1.5 cm in front of the ear mould element (1).
